# EUROPEAN PATENT APPLICATION

(11) **EP 3 056 228 A1**
(43) Date of publication of application: **17.08.2016**
(21) Application number: 16163898.6
(22) Date of filing: 12.09.2013
(51) Int. Cl.: A61L 27/58, B25J 9/00

(54) **PARALLEL KINEMATIC MECHANISM AND BEARINGS AND ACTUATORS THEREOF**

(30) Priority: 12.09.2012 US 201261700080 P
(62) Divisional of application: 13836971.5
(71) Applicant: Memorial University of Newfoundland, Newfoundland and Labrador A1C 5S7 (CA)
(72) Inventor: Krouglicof, Nick, St. John's Newfoundland and Labrador A1C4B1 (CA); Rahman, Taufiqur, St. John's Newfoundland and Labrador A1B1H8 (CA); MacNeil, Levi, Kelligrews Newfoundland and Labrador A1X7K8 (CA); Morgan, Michael, St. John's Newfoundland and Labrador A1B0L4 (CA); Pansare, Nikhil, St. John's Newfoundland and Labrador A1Z1G6 (CA); Hicks, Dion, St. John's Newfoundland and Labrador A1B1G6 (CA); Roberts, Matthew, Portugal Cove St. Philips Newfoundland and Labrador A1M1X3 (CA)
(74) Representative: V.O.

(57) **Abstract**

An improved parallel kinematic mechanism to orient a platform has a higher range of motion for its volume due to the use of magnetically coupled ball joints at the orienting platform and the individual linear actuators operating those joints. The linear actuators may be printed circuit board (PCB) based voice coil actuators, in a magnetic field which may be generated by permanent magnets configured as a modified Halbach array. The PCB based voice coil actuators may have a position sensitive device (PSD) embedded on the PCB to assist in determining location of the actuator with a high degree of accuracy. The payload of the orienting platform may be dynamically repositioned with improved accuracy and speed.

## Description

### FIELD

The present disclosure relates to kinematic mechanisms configured as orienting platforms and the bearings, actuators and controllers thereof.

### BACKGROUND

Electro-mechanically controlled mounting platforms have numerous uses in photography, tracking, robotics, manufacturing and other fields where precision, range of motion, and responsiveness in the manipulation and orientation of a tool are desirable or required.

Limitations to current kinematic devices arise in the range of motion of the bearings, the responsiveness of the actuators, controllers adapted to manipulate improved parts, and the desire for devices tailored to the needs of particular opto-mechatronic applications. Further, existing devices may not meet additional limitations related to the size, weight, power consumption, (i.e.; SWAP requirements), as well as the reliability, of the device which may be demanded by customers for particular applications.

In respect of the bearings. Conventional three degree-of-freedom (spherical) ball joints and rod-end bearings have three major shortcomings: (1) they offer a limited range of angular motion, (2) significant backlash is often present which adversely affects accuracy, and (3) there is often appreciable friction which adversely affects the dynamic performance.

These shortcomings limit the utility of conventional ball joints in small, precision parallel kinematic mechanisms (PKMs). This is particularly true in opto-mechatronic applications where range of motion, accuracy, and dynamic response are critical. For example, in a typical, small rod-end bearing, the range of angular motion is limited to 20 degrees; and commercially available ball joints have a range of motion typically limited to 35 degrees. The available range of motion is a serious limitation in the design of parallel kinematic mechanisms for opto-mechatronic application including active vision systems where the camera is actively directed at a region of interest.

Backlash is also a major problem with commercially available ball joints. In existing systems, tension springs may be used to pre-load the rod end bearings in an effort to minimize the backlash. The addition of springs increases the likelihood of link interference.

In respect of the actuators, voice-coil (i.e., linear electric) actuators are simple electromechanical devices that generate precise forces in response to electrical input signals. Fundamentally they are the simplest form of electric motor consisting of a non-commutated single coil or winding moving through a fixed magnetic field (which may be produced by stationary permanent magnets). From a system design point of view, it is generally the end-user's responsibility to couple the voice-coil actuator with a linear bearing system, position feedback device, switch-mode or linear servo amplifier, and motion controller. The integration of multiple discrete components adversely affects system reliability and renders minimization and packaging difficult particularly when multiple actuators are required. The moving mass of the voice-coil actuator is also often a design limitation. There is a desire in parallel kinematic mechanisms, particularly in opto-mechatronic applications, to improve the dynamic response and precision of the actuators by both reducing the moving mass of the actuator, increasing the ratio of force to electrical current (i.e.; the force constant) and increasing the range of motion.

In respect of prior kinematic structures (i.e., well-defined arrangements of links and joints) that can achieve spherical motion of a payload, certain of these are capable of delivering high accuracy and dynamics, thanks to their parallel arrangements of the links and joints. However, these other designs demonstrate lower load carrying capacity, slower dymanic response, lower accuracy, and are unable to achieve the large range of motion within a small volume.

A prototype of an orienting platform based on similar kinematics was constructed by Thomas Villgrattner et al of Technical University of Munich. However, without the linear actuators and improved ball joints disclosed herein, range of motion and dynamic response was limited.

A generalized controller algorithm proposed by Jingqing Han, and summarized in "From PID to Active Distrubance Rejection Control", IEEE Trans. Ind. Elec., Vol. 56, No. 3, March 2009, pp 900-906, is applicable for tracking control of any dynamic system, and has been used in some instance for kinematic mechanisms. However, in a research paper published in the IEEE Transactions on Control Systems Technology (Vol 12, No 3, May 2004) it was reported that while this controller algorithm was implemented in software using a PC for the tracking control of a Stewart Platform (paper title: Disturbance-Rejection High-Precision Motion Control of a Stewart Platform), the controller was not fast enough to utilize the available dynamics of the actuators.

### SUMMARY

A number of improvements in parallel kinematic mechanisms and their constituent parts are now disclosed.

In respect of the bearings: the magnetically-coupled ball joint proposed and described herein is an assembly of three components: (1) A small, typically cylindrical permanent magnet (e.g. using neodymium; the strongest permanent magnet currently available); (2) A ball manufactured from a ferrous material (e.g. a ferrous stainless steel), and incorporating a cylindrical rod that can be used to secure the ball to the mechanism of interest; and (3) A socket/base/separator with a magnet on one side, and a cavity shaped like a section of a sphere to accept the ball on the other. In one example, the socket/base/separator is manufactured out of polyoxymethylene (also known as acetal, polyacetal, and polyformaldehyde), an engineering thermoplastic used in precision parts to provide high stiffness, low friction and dimensional stability.

In respect of the actuators, a low-inertia voice-coil design is described whereby the traditional moving coil is replaced with a Printed Circuit Board (PCB) that incorporates the necessary windings as traces on the board. Control of the actuator requires precise information on coil position. In one example, the position feedback device, specifically a one-dimensional Position Sensitive Device (PSD) may be incorporated directly on the PCB. Various tolerances for the position control may be determined by design. Position resolution on the order of a micron, as well as signal conditioning and motion control electronics, can be integrated on the linear actuator, including (but not necessarily) on the same PCB using Surface-Mount Technology (SMT).

Typical applications of the PCB based voice coil with integrated position sensing electronics include, but are not limited to: (1) Linear motor for operating a single prismatic mechanism; (2) use in a Parallel Kinematic Mechanism (PKM), wherein multiple actuators of the above type can control multiple bearing elements that can move and orient a payload; and (3) calibration of Micro-Electro-Mechanical Systems (MEMS) sensors.

The following features, individually and collectively, differentiate the PCB based voice coil with integrated position sensing electronics: (1) fabrication of the "windings" of the voice coil as conductive traces on a PCB facilitates manufacture, reduces mass, and provides a working medium for other device elements; (2) use of multiple layers of windings on the same PCB increases the force constant of the actuator; (3) use of a modified Halbach array of permanent magnets generates a stronger magnetic field about the planar PCB based windings while reducing the magnetic field on the exterior of the actuator; (4) Integration of a 1 D Position Sensitive Detector (PSD) or other feedback device onto the PCB of the voice-coil actuator simplifies mass production of an operable mechanical unit and eases integration into other systems; and (5) the ability to incorporate signal conditioning and motion control electronics on the PCB further improves the robustness of the design and facilitates adoption where linear actuators are desired.

Using the above noted PCB based linear actuator operating the above noted magnetically couple ball joint, a parallel kinematic mechanism can move and orientate a payload in up to six degrees of freedom (in the figures shown, the PKM is limited to 3 degrees of freedom) with fast dynamic response, high precision, and high reliability. In the intended applications, the payload can take the form of a camera, a laser, mirror, antenna, range finder, communications device, optical assembly (e.g.; telescopic sight), or a sensor that must be pointed and/or moved in different directions. The PKM disclosed herein is a pointing device that is capable of orientating a payload in three degrees of rotational freedom with high accuracy. In addition, the kinematic mechanism provides very fast movements and features a high ratio of motion range to physical volume of the prototype. Unlike a conventional pointing device (e.g., a gimbal mechanism), this PKM employs three linear actuators to achieve spherical motion (i.e., motion about a fixed center) of the payload.

The resulting spherically, orienting platform may be used in a number of applications, including, but not limited to: (1) for smart laser scanning (i.e., selective scanning for minimization of extraneous data) of a remote environment: using the pointing device to manipulate the laser; (2) tracking and filming of fast moving objects by a camera mounted on the pointing device; (3) time of flight range finding system: pointing a laser range finder at a fast-moving target such as an aircraft, and (4) for implementing free space optical communications (FSOC).

In one example, an improvement herein disclosed is an orienting platform for carrying a payload connected to one or more links operated by a voice coil linear actuator. In another example, the links include at least one spherical layer formed by a magnetically coupled rod end bearing. In yet another example, the PKM comprises a platform having 3 links, each operated by a PCB based voice coil actuator, the links being formed of a prismatic joint (the actuator), a spherical joint on the actuator, connected by a rod to a spherical joint on the orienting platform. The orienting platform is further connected by a seventh spherical joint to a fixed point within the PKM. The spherical joints are preferably of the magnetically coupled ball joint/rod end bearing variety disclosed herein. The actuator is preferably of the PCB based voice coil linear actuator variety disclosed herein. Linear motion of the actuators is translated to angular motion of the orienting platform according to relationships based on the parallel kinematics of the design.

A digital controller based on the generalized algorithm of Jingqing Han, is implemented for the parallel kinematic mechanism and the linear actuators. Implementation of the controller using parallel computing methods and/or a hardware implementation in an electronic device such as a Field-Programmable Gate Array (FPGA), complex programmable logic device (CPLD), etc., overcomes prior deficiencies in achieving controller response times on the order of the actuator response times.

### BRIEF DESCRIPTION OF THE DRAWINGS

Figure 1 shows a partial transparent view of one embodiment of the pre-loaded, magnetic rod end bearing.
Figure 2 shows an exploded side view of the magnetic rod end bearing of Figure 1, highlighting the separation distance between the magnet and the ferrous ball.
Figure 3 is a perspective view of a diagram of one example of the PCB based voice coil actuator.
Figure 4 is an exploded perspective view of an example PCB based voice coil linear actuator of Figure 3 (cabling, rivets and connectors removed).
Figure 5 is a top view photograph of another PCB board designed for use with a linear PCB based voice coil actuator of the current disclosure, showing windings etched in a double rectangular spiral left and right on the board on one or more layers of the PCB.
Figure 6 is a magnetic flux diagram of a modified Halbach array adapted for use with the windings of the PCB board design shown Figure 5.
Figure 7 is a top view photograph of another configuration of windings on the PCB board designed for use with a linear PCB based voice coil actuator of the current disclosure, showing windings etched in a rectangular spiral pattern on the printed circuit board, which pattern may be repeated on multiple layers within the PCB.
Figure 8 is a magnetic flux diagram of a modified Halbach array adapted for use with the windings of the PCB board design shown Figure 7.
Figure 9 is a perspective view of a diagram of a parallel kinematic mechanism orienting/pointing device, with the actuators in a full triangular configuration, and the orienting platform in a plane perpendicular to the motion of the actuators.
Figure 10 is a top view of the orienting/pointing device of Figure 9.
Figure 11 is a side view of the orienting/pointing device of Figure 9.
Figure 12 is a perspective view of a diagram of a parallel kinematic mechanism orienting/pointing device, with the actuators in a Y- configuration.
Figure 13 is a perspective view of a diagram of a parallel kinematic mechanism orienting/pointing device, with the actuators in a skew configuration.
Figure 14 shows a line drawing schematic of the 3-P-S-S/S architecture of a modelled PKM on the left side and a 3-D representation of the line drawing on the right side.
Figure 15 shows an second perspective of the parallel kinematic mechanism orienting/pointing device of Figure 13.
Figure 16 is an exploded perspective view of another example of a PCB based voice coil linear actuator having a laser diode position sensor.

### DETAILED DESCRIPTION

One or more preferred embodiments of the parallel kinematic device of the present disclosure will now be described in greater detail with reference to the accompanying drawings.

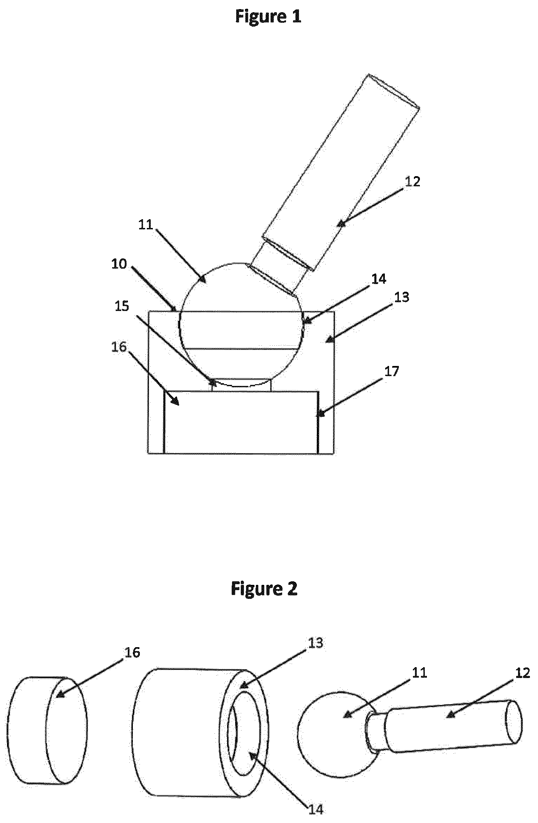

Figures 1 and 2 relate to improvements in bearings or ball joints.

Figure 1 shows one example of the rod end bearing **10** having: a ferrous spherical ball **11** connected with a rod **12** which could be affixed to some further link that rotates about the bearing (not shown); a magnet **16** to retain the spherical ball **11**; and a base/socket/separator **13** with a spherical section shaped cavity **14** to fit the ball 11 and to provide a low friction separation between the ball **11** and the magnet **16**. An appropriately shaped cavity **17** may also be provided to fit the **16**, A space **15** separates the ball 11 and the magnet **16**, which by design is small enough to permit the joint to stay connected in use.

Since the spherical ball **11** is held securely in place by the magnet **16**, the mating spherical cavity **14** in the base **13** can be made smaller than a half sphere which provides motion in excess of 180 degrees in all three degrees-of-freedom. The magnet also effectively preloads the joint **10** thereby reducing the backlash to zero without the use of external springs. Finally, a low friction surface of the base **13** in contact with the ball is desirable, and so when the entire base is made of polyoxymethylene, there is an inherent low friction surface for the spherical ball **11**. When the base **13** is not made of polyoxymethylene, another self-lubricating or low friction surface should be used between the separator and the ball. A design trade-off can be made between the friction force holding the mechanism together and the force required to separate the ball from the base, by adjusting the separation distance **15** between the cavity for magnet **17** and the bottom of the cavity for the spherical ball **14**. Figure 2 shows another view of the parts disassembled.

The ball and rod assembly **10** may be manufactured as one piece on a precision, Computer-Numerically Controlled (CNC) lathe. An alternative is to purchase a precision tooling ball that has the same overall shape (tooling balls are frequently used in mechanical metrology). The spherically shaped cavity between the base (separator element) and the ball can also be manufactured on a precision, CNC lathe. The exact outer shape of the base can be modified to facilitate integration in the mechanism of interest and a cavity for the magnet may also be provided, but is not crucial.

In this fashion, the magnetically coupled ball or spherical joint **10** of the current disclosure offers certain possible advantages: (1) large range of angular motion; (2) near zero backlash due to inherent preloading of the joint by design; (3) low friction and/or (4) control of pre-load friction as a parameter. A joint with these characteristics has applications in the design of parallel kinematic mechanisms (PKMs) with a wide range of motion, high accuracy and repeatability, and fast dynamic response. PKMs are becoming increasingly popular in opto-mechatronic applications. Because of their unique kinematic structure, PKMs are capable of delivering high dynamics with low encumbrance while maintaining favorable stiffness characteristics and superior functional accuracy. Despite these advantages, one of the main limiting factors that has hindered their wide spread use is the available range of angular motion of the joints - which the current magnetically coupled ball joint helps address.

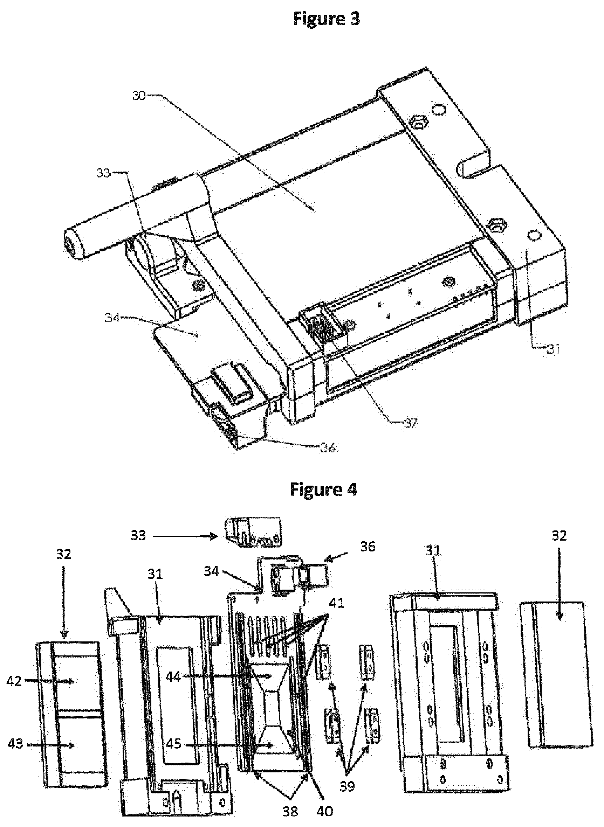

Figures 3, 4, 5, 6, 7 and 8 relate to improvements in a voice coil actuator, which may be used in various applications, including a parallel kinematic orienting device as further discussed in the examples below.

Figure 3 is a perspective view of a diagram of the PCB based voice coil actuator **30** in which the housing **31** orients the magnets **32** so as to create a strong magnetic field across windings on a PCB **34** located within a cavity of the housing **31** which permits linear motion of the PCB **34**. When a current flows through the windings of the PCB **34**, the magnetic field created by the magnets **32** generates a force on the PCB **34**. Using an appropriate magnetic field and windings permits the PCB 34 to be accelerated linearly within the magnetic field created. Control electronics **36** on the PCB **34** are to be connected to control electronics **37** on the housing **31**, to provide current to the winding and to power and communicate with other onboard surface mounted technology, not shown in this example.

Figure 4 is a exploded perspective view of the PCB based voice coil actuator **30** of Figure 3. Sets of permanent magnets are configured in modified Halbach arrays **32** on opposite sides of the housing **31**. Rails **38** running in rail guides **39** assist in maintaining the linear motion of the PCB **34** within the housing, in a forward or up direction towards the armature **33** or a backwards or down direction on the bottom. Control electronics **36** on the PCB **34** provide power to the windings **40**. The current in the forward windings **44** interacts principally with the magnetic field from the forward set of magnets **42** in the modified Halbach array **32**. Since the current in the backwards windings **45** flows in the opposite direction, and interacts principally with the magnetic field from the backward set of magnets **43**, the force generated from the windings is doubled up for the same amount of current. Slots **41** in the PCB 34 may allow for additional structural elements (such as magnet separators or additional guides or stops, not shown) to be included in the design.

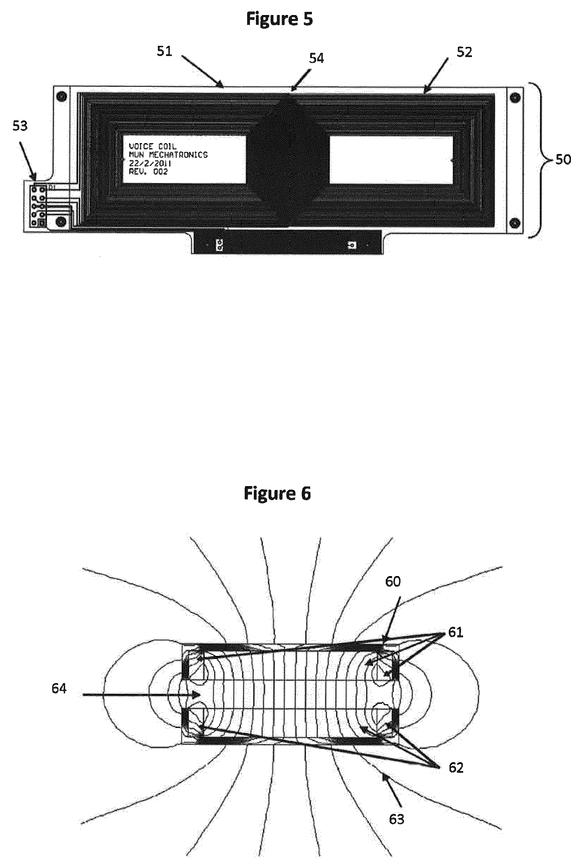

Figure 5 shows one example of a configuration of windings on the PCB **50** in which two sets of windings **51, 52** located to the left and right of the center line of the PCB **50**, respectively, define a middle set of conductive traces **54** (conductors) in which current can be caused, by circuitry connected to the connectors **53** to flow in the same direction.

Figure 6 shows one arrangement of permanent magnets **61, 62**, within the housing **60** of a PCB based voice coil linear actuator above and below a channel **64** for the PCB as a modified Halbach array chosen to operate with the winding configuration of Figure 5. The magnetic field **63** is predominantly in a single direction across the middle set of conductors **54** of Figure 5, throughout the range of motion of that PCB. A current in a clockwise direction in the left set of windings **51** and a current in a counter clockwise direction in the right set of windings **52** will appear in the middle set of conductors **54** as current travelling in the same direction within the magnetic field **63**, which will induce a force in one direction. Switching the direction of the current in both sets of windings **51, 52**, will induce a force in the other direction. Relative motion between the PCB **50** and the housing **60** is measured with one or more PSDs integrated within the linear actuator itself. In one embodiment, the static magnetic field in the immediate, exterior vicinity of the device may be reduced by orienting the permanent magnet in a Halbach magnetic array. This provides a strong, substantially uniform interior magnetic field while ensuring the exterior field is near zero. Shielding is used to further decrease the magnetic field outside the device.

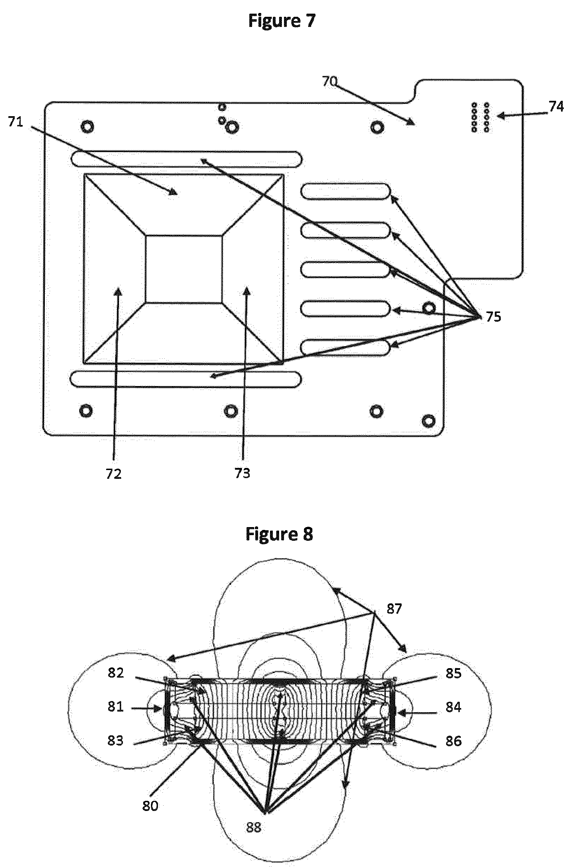

Figure 7 shows another possible configuration of the windings/coil on a PCB **70** in which a single rectangular spiral pattern of tracings for the winds of the coil **71** (albeit, possibly in one or more layers within the PCB itself) is used. Current in the region **73** towards the slots **75** flows in one direction while current in the region **72** away from the slots **75** flows in the other direction. In order to take full advantage of the available length of conductor within the magnetic field, the magnetic field in the vicinity of region **72** and region **73** must be opposite over the operating range of the device. Figure 8 shows a method of configuring the permanent magnets **82, 83, 84, 85, 86, 88** within the housing **80** as a modified Halbach array in which two opposing sets of 5 magnets each are used to generate the magnetic field **87**. Large plane magnets **82, 83, 85, 86** corresponding to the regions **72** and **73** of the conductors in Figure 7 have opposite poles facing the windings/tracings **71**. Smaller magnets **88** separate them with their poles orienting the magnetic flux between the larger magnets, and smaller magnets 88 on each end also direct the magnetic flux **87** back into the regions **81** and **84** of the modified Halbach array in the housing 80, corresponding to regions **72** and **73** of the PCB 70 for which it is designed..

Where more than one layer of windings are present within the PCB, it is important for the stacked layers to have the same orientation (clockwise versus counterclockwise), and as such, if one layer spirals inward, the next layer (from the perspective of the conductive trace) spirals outward.

In this fashion, the reversing magnetic field as between regions **81** and **84** allows two different sections **72 73** of the winding **71** to generate a force in the same direction thereby doubling the force constant of the actuator. In Figure 7, the current in region **72** of the coil 71 is opposite in direction to the current in region **73** of the coil **71.** However, since the Halbach array of Figure 8 reverses the direction of the magnetic field between the two corresponding regions **81** and **84**, the force generated by both sections acts in the same direction. In this configuration of the windings and magnets of Figures 7 and 8, the increased strength of the magnetic field combined with the increased length of conductor within the magnetic field effectively increases the force constant by a factor of four compared to the example of the PCB in Figure 5 and the magnetic field of Figure 6, in which only the middle set of conductors generated a force. This example from Figures 5 and 6; however, is more suitable for long stroke (i.e.; range) applications. For example, a linear actuator using the PCB **50** and magnet array **60** depicted in Figures 5 and 6 could have an three times the effective range compared to a comparable device using the PCB **70** and magnet array **80** of Figures 7 and 8. The latter device generates approximately four times the force for the same current and length of conductor, but at the expense of range of operation. This is in part due to using two sides of the same winding and in part due to the preferred configuration of the magnets.

Other configurations are possible, including pluralities of layers of windings. Electronics may be mounted on the board. Electromagnets may be used in place of the modified Halbach arrays.

Analysis and experimental investigation suggests that based on a three ounce copper PCB with 150 micron traces/spaces and a voice-coil stroke of 37 mm for the PCB of Figure 5, the dynamic performance parameters of the actuator (e.g., coil inertia, force constant, maximum velocity) are superior to those of commercially available products. A three ounce copper PCB with 150 micron traces/spaces and a voice-coil stroke of 12 mm for the PCB of Figure 7 shows approximately four times the force for the same current

Some features of a linear actuator using the PCB based voice coil design which distinguish it from commercial devices performing a similar function, are: (1) replacement of the traditional moving coil with a PCB that incorporates the necessary windings as conductive traces on one or more layers of the board. The PCB has low moving mass, is easy to mass-produce, and is compact. The coil is in a planar orientation relative the applied magnetic field; (2) this permits use of a planar magnetic field across a housing, which may also take advantage of opposing Halbach magnet arrays to provide a strong internal magnetic field while minimizing the external field; (3) integration of a one-dimensional Position Sensitive Device (PSD) on the PCB of the actuator to provide accurate position feedback for motion control. A PSD is noncontact, highly accurate and has a fast response time; and (4) Incorporation of signal conditioning and motion control electronics on the PCB containing the traces.

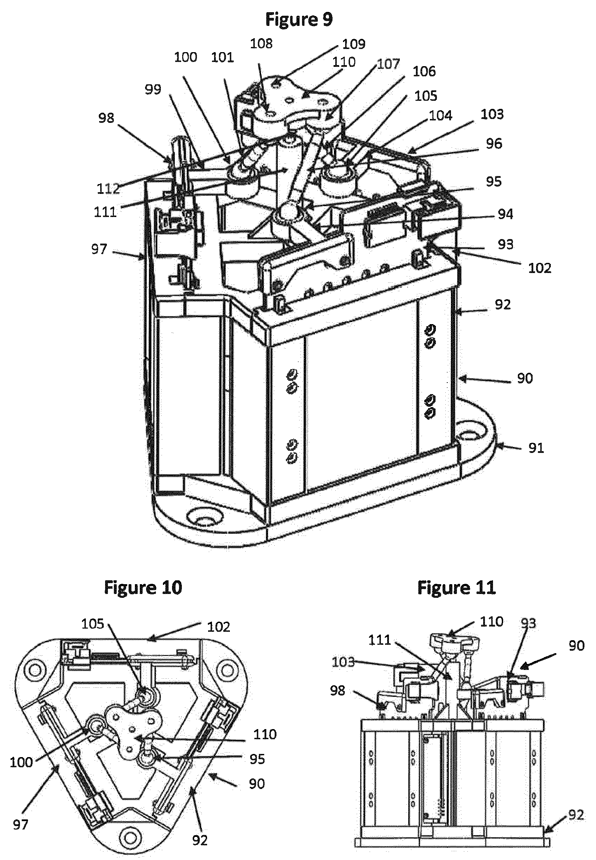

Figures 9, 10 and 11 show the parallel kinematic mechanism **90** in which three linear actuators **92, 97, 102** (based on the designs discussed above) are used to drive/control an orienting platform **110.** To preserve a common pivot point, the orienting platform **110** is connected by a spherical ball joint/bearing **112** (usefully the magnetically coupled rod end bearings of the above design, but not necessarily) at a fixed height relative to the housing **91** by a central pillar/link **111.** The motion of the PCBs **93, 98, 103** on the three linear actuators **92**, **97, 102** drive independent links comprised of the armatures having magnetically coupled rod end bearing bases **94, 99, 104**, to receive ferrous balls **95, 100, 105**, connected to the rods **96**, **101, 106**, respectively. In the example shown, the spherical links are implemented using the magnetically coupled rod end bearings of the type shown in Figures 1 and 2. The connections of each rod **96, 101, 106** to the orienting platform **110** are completed by more magnetically coupled rod end bearings **107, 108** and **109**. As can be inferred from the Figures, driving each of the linear actuators **92, 97 and 102** will have the effect of tipping the plane of the orienting platform in a different direction. The housing **91** of the PKM **90** is configured to join the linear actuators **92, 97** and **102** in a large triangle.

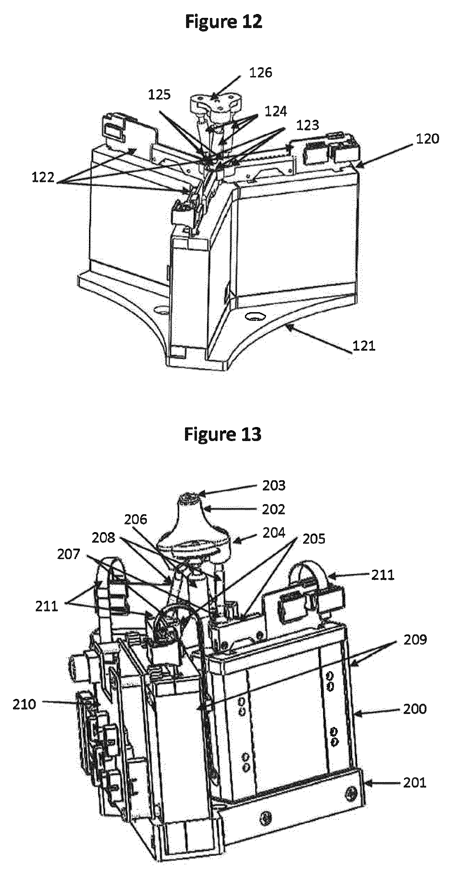

Figure 12 shows another example configuration of the parallel kinematic mechanism **120** controlling an orienting platform **126** in which the housing **121** for the linear actuators configures them in a Y-shape. Armatures **123** directly on the printed circuit boards **122** connect by magnetically coupled ball joints to the rods **124**, which connect by more magnetically coupled ball joints to the orienting platform **126**.

Figure 13 is yet another example configuration of the parallel kinematic mechanism **200** in which the base **201** is designed to configure the voice coil linear actuators **209** in a skewed pattern forming a mini-triangle. A laser, camera or other instrument **203** is affixed to a mount **202** on the mounting/orienting plate **204**. Ball joints **207** and rods **208** connect the mounting/orienting plate **204** to the armatures **205**. The armatures **205** do not need to be as long as in the example of Figure 9, and can be more centrally positioned over voice coil actuator **209** than in the example of Figure 12. The centre pole **206** from the housing **201** connects to a ball joint **207** on the mounting/orienting platform **204**. Flexible cables **211** connect each voice coil actuator **209** to an onboard controller **210**.

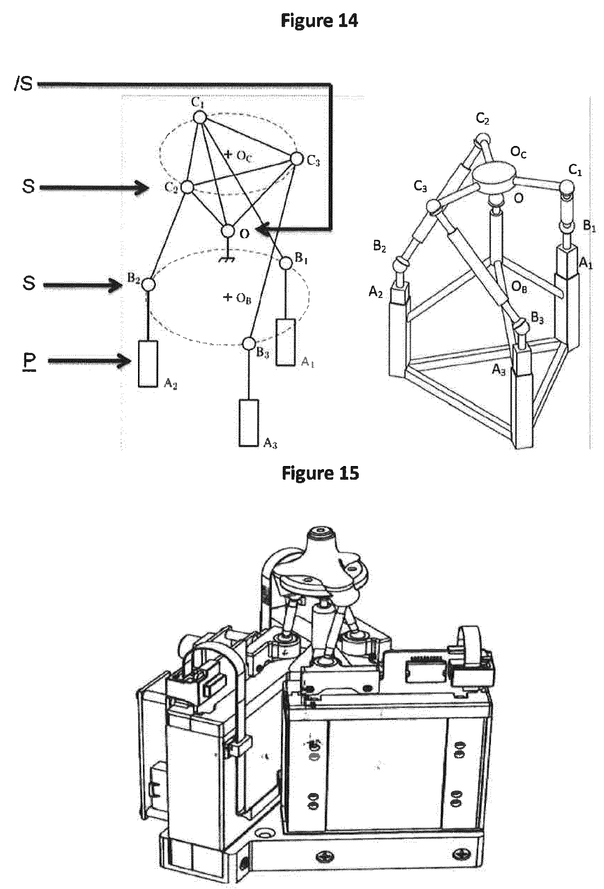

Figure 14 shows a representation of the PKM as prismatic actuators (**A₁, A₂, A₃**) forming a prismatic layer (**P**), to operate spherical bearings (**B₁, B₂**, **B₃**) in a first spherical layer (S - **O_{B}**), which due to the linkages, force corresponding motion in spherical bearings (**C₁, C₂, C₃**) in a second spherical layer (S - **O**_{C}), further restricted by the fixed spherical link (**O**) representing a third spherical layer (/S). The right hand image shows a more detailed 3 dimensional rendering in which linkages between the first spherical layer (S - **O**_{B}) and the second spherical layer (S - **O**_{C}) can be envisaged as incorporating the magnetically coupled base while the prismatic actuators and the fixed spherical link (**O**) (corresponding to the platform), provide the ferrous balls to for the ball joints.

Figure 15 shows another perspective of the PKM of Figure 13 with the labelling of parts removed.

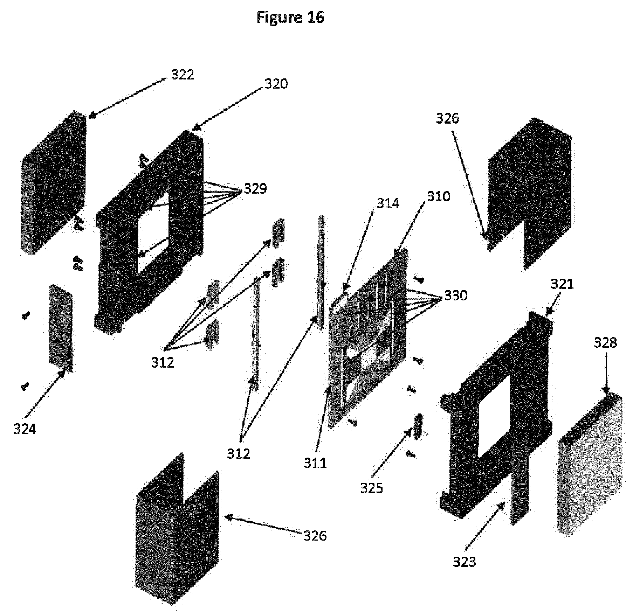

Figure 16 is an exploded perspective view of another one example of the linear actuator, in which the voice coil PCB **310** is provided with a laser diode **311** on the front and linear bearing guide rails **312** on the back. A power connection **314** is also provide to power the voice coil PCB **310** and the laser diode **311**. Front and back are orientations provided for convenience of describing the design only, and are not limitations. The housing of the linear actuator is comprised of a back magnet holder **320** to hold a back magnet **322** and a front magnet holder **321** to hold a front magnet **328**. The guide rails **312** of the voice coil PCB **310** are capable of sliding within linear bearing carriages **313** of the back magnet holder **320**. The laser diode **311** triggers the position sensitive detector **325** on the front magnet holder **321.** Other position sensitive arrangements may be used, but this configuration provides the potential for micron level position accuracy which leads to greater accuracy in angular orientation of bears operated by the linear actuator. Other electronics may also be provided directly on the linear actuator, including a power PCB **324** and a signal conditional PCB **323**. Once assembled, steel jacket elements **326** encapsulate the magnet holders to further shield and direct the magnetic flux.

Due to the strong magnetic fields across the modified Halbach array of magnets **322** and **328** on each face of the PCB board, there is a tendency for the actuator housing to deform. To counteract this deformation, slots **330** over the full range of motion may be cut into the PCB board and spacing elements **329** between the front magnet holder **321** and back magnet holder **320** fitted through the slots can effectively prevent large deformations which might interfere with the motion of the PCB. This feature of the voice coil PCB of Figure 16 is also shown in Figure 7.

The foregoing examples and advantages are merely exemplary and are not to be construed as limiting the present invention. The present teaching can be readily applied to other types of apparatuses. Also, the description of the examples of the present inventions is intended to be illustrative, and not to limit the scope of the claims, and many alternatives, modifications, and variations will be apparent to those skilled in the art.

## Claims

1. An parallel kinematic mechanism comprising:
a. an orienting platform connected by one or more links to a housing;
b. at least one of the links controlled by a PCB based voice coil linear actuator.

2. The parallel kinematic mechanism of claim 1 in which the one or more links include a magnetically coupled ball joint.

3. The parallel kinematic mechanism of claim 1 in which:
a. The orienting platform is fixed to the housing by a central ball joint and controlled by three control links; and
b. Each control link comprises a PCB based voice coil linear actuator as a prismatic joint connected by a first ball joint to a rod, the rod connected by a second ball joint to the orienting platform.

4. The parallel kinematic mechanism of claim 3 in which the central ball joint and the first ball joints and second ball joints on each control link are magnetically coupled ball joints.

5. The parallel kinematic mechanism of claim 4 in which each magnetically coupled ball joint is comprised of a ferrous ball in a low friction polyoxymethylene base held in place by a neodymium magnet.

6. The parallel kinematic mechanism of claim 1 in which each PCB based voice coil linear actuator comprises:
a. An actuator housing defining a channel for a printed circuit board to move in a direction perpendicular to one or more sets of tracings etched to form one or more coils on the printed circuit board;
b. The one or more sets of tracings connected to control circuitry capable of delivering a current to the one or more coils;
c. A magnetic field across the channel to induce a force on the printed circuit board that is proportional to current in the one or more coils; and
d. A position sensitive device on the printed circuit board connected to the control circuitry and operable therewith to communicate changes in the position of the printed circuit board to the control circuitry.

7. The parallel kinematic mechanism of claim 6 in which, for each PCB based voice coil linear actuator:
a. the magnetic field forms a first active region and a second active region in which the magnetic field flows in predominately opposite direction to the first active region, and
b. the tracings are configured in a single rectangular spiral on one or more layers of the printed circuit board such that current in the tracings within the first active region flows in a direction opposite to current in the tracings within the second active region.

8. The parallel kinematic mechanism of claim 7 in which, for each PCB based linear actuator, the magnetic field is generated by permanent magnets configured as Halbach arrays on each side of the channel.
